# EUROPÄISCHE PATENTSCHRIFT

(11) **EP 3 997 063 B1**
(45) Veröffentlichungstag und Bekanntmachung des Hinweises auf die Patenterteilung: **02.08.2023**
(21) Anmeldenummer: 20735518.1
(22) Anmeldetag: 25.06.2020
(51) Int. Cl.: C07C 277/08, C07C 279/14, A23K 20/142

(54) **VERWENDUNG EINER METASTABILEN KRISTALLMODIFIKATION UND VERFAHREN ZU DEREN HERSTELLUNG**
USE OF A METASTABLE CRYSTAL MODIFICATION AND METHOD FOR THE PRODUCTION THEREOF
UTILISATION D' UNE MODIFICATION CRISTALLINE MÉTASTABLE ET PROCÉDÉ DE PRODUCTION ASSOCIÉ

(30) Priorität: 12.07.2019 DE 102019118894; 12.07.2019 DE 102019118893
(43) Veröffentlichungstag der Anmeldung: 18.05.2022
(73) Patentinhaber: Alzchem Trostberg GmbH, 83308 Trostberg (DE)
(72) Erfinder: GÜTHNER, Thomas, 83308 Trostberg (DE); THALHAMMER, Franz, 83308 Trostberg (DE); SANS, Jürgen, 83308 Trostberg (DE)
(74) Vertreter: Weickmann & Weickmann PartmbB
(86) Internationale Anmeldenummer: PCT/EP2020/067838
(87) Internationale Veröffentlichungsnummer: WO 2021/008844

(56) Entgegenhaltungen:
- CN-A- 101 525 305
- DE-C- 964 590
- US-A- 2 654 779
- G. P. JONES ET AL: "Conformations of GABA analogues. I: Crystal and molecular structure of guanidinoacetic acid", JOURNAL OF CRYSTAL AND MOLECULAR STRUCTURE, Bd. 9, Nr. 5, 1. Oktober 1979 (1979-10-01) , Seiten 273-279, XP055716473, US ISSN: 0308-4086, DOI: 10.1007/BF01320842
- S. GUHA: "The crystal and molecular structure of glycocyamine", ACTA CRYSTALLOGRAPHICA. SECTION B, STRUCTURAL CRYSTALLOGRAPHY AND CRYSTAL CHEMISTRY., Bd. 29, Nr. 10, 1. Oktober 1973 (1973-10-01), Seiten 2163-2166, XP055716475, DK ISSN: 0567-7408, DOI: 10.1107/S056774087300628X

## Beschreibung

Die vorliegende Erfindung betrifft die Verwendung einer Kristallmodifikation von N-(Aminoiminomethyl)-2-aminoethansäure zur Herstellung weiterer Produkte und sowie ein Verfahren zur Herstellung dieser Kristallmodifikation.

N-(Aminoiminomethyl)-2-aminoethansäure (CAS-Nr. 352-97-6, Summenformel C₃H₇N₃O₂), auch als Guanidinoessigsäure, Guanidinoacetat, Glycocyamin, N-Amidinoglycin oder N-(Aminoiminomethyl)-glycin bekannt, ist eine Guanidinocarbonsäure mit vielfältigen Anwendungen, u.a. in der Synthese von chemischen Produkten, insbesondere Pharmazeutika (vgl. WO 2000/059528), zur direkten Anwendung als pharmazeutischer Wirkstoff bei Nierenerkrankungen (vgl. JP 60054320) oder neurodegenerativen Erkrankungen (vgl. CN 106361736), in der Herstellung von Polymeren (vgl. Du, Shuo et. al., Journal of Materials Science (2018), 53(1), 215-229), als Komplexbildner für Metalle (vgl. Lopes de Miranda et.al., Polyhedron (2003), 22(2), 225-233 bzw. Singh, Padmakshi et. al, Oriental Journal of Chemistry (2008), 24(1), 283-286) und als Zusatzstoff für die Ernährung von Tieren, insbesondere Säugetieren, Fischen, Vögeln (vgl. WO 2005/120246) und dem Menschen (vgl. WO 2008/092591, DE 102007053369).

N-(Aminoiminomethyl)-2-aminoethansäure lässt sich z.B. nach Strecker, M. (Jahresber. Fortschr. Chem. Verw. (1861), 530) aus Glycin durch Umsetzung mit Cyanamid herstellen. Alternativ kann N-(Aminoiminomethyl)-2-aminoethansäure z.B. durch Umsetzung von Glycin mit S-Methylisothioharnstoff-Jodid unter Verwendung von Kaliumhydroxid als Base hergestellt werden (vgl. US 2,654,779). Auch die Umsetzung von Chloressigsäure mit Ammoniak zu Glycinhydrochlorid und dessen weitere Umsetzung mit Cyanamid wurden beschrieben (vgl. US 2,620,354). Die Umsetzung von Cyanamid mit Gylcin zu Glycocyamin bei einem pH-Wert von 9 bis 10 wurde in DE 964 590 beschrieben.

G.P. Jones und P. J. Pauling, Journal of Crystal and Molecular Structure, Vol. 9, No. 5, 1979, 273-279 beschreiben eine Kristall-und Molekularstruktur von Guanidinoessigsäure. S. Guha, Acta Cryst. (1973), B29, 2163-2166 beschreibt eine Kristall- und Molekularstruktur von Glycocyamin.

Bei den bekannten Verfahren fällt N-(Aminoiminomethyl)-2-aminoethansäure als feinkristallines Pulver an, welches einen erheblichen Staubanteil, d.h. einen erheblichen Anteil an Partikeln, die eine Korngröße kleiner 63 µm besitzen, aufweist.

Für die Handhabung von chemischen Produkten in fester Form ist es oftmals wünschenswert, dass diese in kristalliner, körniger, rieselfähiger, staubfreier Form ohne oder mit nur geringem Feinkornanteil vorliegen. Insbesondere für die Anwendung als Futtermittelzusatzstoff ist ein schlecht rieselfähiges, staubendes Pulver völlig ungeeignet.

Um diesem Sachverhalt zu begegnen, wurde beispielsweise vorgeschlagen, N-(Aminoimino-methyl)-2-aminoethansäure unter Zusatz von polymeren Bindemitteln (z.B. Methylcellulose) in Mengen von 0,05 bis 15 Gew.-% und Zusatz von Wasser zu Formlingen, Granulaten oder Extrudaten umzuformen (vgl. WO 2009/012960). Nachteilig an diesem Verfahren ist, dass ein Zusatz eines Fremdstoffs, nämlich eines Bindemittels, zwingend erforderlich ist, und dass in einem zusätzlichen Verfahrensschritt, unter Verwendung eines speziellen, technisch aufwendigen und teuren Apparats, wie beispielsweise einem Extruder, Granulator, Intensivmischer oder Pflugscharmischer, und nachfolgender Trocknung das Granulat bzw. die Formlinge hergestellt werden müssen.

Nachteilig am Verfahren gemäß obigem Stand der Technik ist zudem, dass Formlinge oder Granulate entweder einen hohen Bindemittelanteil und damit eine geringe Lösungsgeschwindigkeit aufweisen, oder aber bei einem geringen Bindemittelanteil sich zwar relativ schnell auflösen, zugleich aber eine geringe Festigkeit und hohe Abriebwerte aufweisen, so dass die Staubfreiheit nicht mehr gewährleistet werden kann.

Der Erfindung liegt daher die Aufgabe zugrunde, ein Verfahren zur Herstellung von N-(Aminoiminomethyl)-2-aminoethansäure in Form von rieselfähigen, nicht staubenden Kristallaggregaten zur Verfügung zu stellen, welche die Nachteile des Standes der Technik nicht aufweisen, sondern einfach und mit verbreiteten Standardapparaten der chemischen Industrie herstellbar sind, und die zudem eine hohe Löslichkeit aufweisen.

Gelöst werden diese Aufgaben durch ein Verfahren zur Herstellung einer thermodynamisch metastabilen Kristallmodifikation von N-(Aminoiminomethyl)-2-aminoethansäure gemäß Anspruch 1. Bevorzugte Ausführungsformen der Erfindung sind in den Unteransprüchen angegeben, die wahlweise miteinander kombiniert werden können.

Das Auftreten von chemischen Stoffen in verschiedenen Kristallformen bzw. Kristallmodifikationen (Polymorphie) ist sowohl für die Herstellung und Anwendung der Stoffe als auch für die Entwicklung von Formulierungen von großer Bedeutung. So unterscheiden sich die verschiedenen Kristallmodifikationen einer chemischen Verbindung neben dem Aussehen (Kristallhabitus) auch in zahlreichen weiteren physikalischen oder physiko-chemischen Eigenschaften. Es ist bisher nicht möglich, das Auftreten und die Anzahl von Kristallmodifikationen einschließlich ihrer physikalischen oder physiko-chemischen Eigenschaften vorherzusagen. Vor allem die thermodynamische Stabilität und auch das unterschiedliche Verhalten nach Darreichung in lebenden Organismen lassen sich nicht im Voraus bestimmen.

Unter gegebenen Druck- und Temperaturbedingungen haben verschiedene polymorphe Kristallmodifikationen üblicherweise unterschiedliche Gitterenergien bzw. Standardbildungswärmen. Die Kristallform mit der niedrigsten Energie wird als stabile Form bezeichnet. Formen mit höherer energetischer Lage werden, sofern sie isoliert werden können, als metastabil (unter den gegebenen Druck- und Temperaturbedingungen) bezeichnet. Metastabile Polymorphe haben die Tendenz, sich in das stabile Polymorph umzuwandeln. Dies erfordert aufgrund der Metastabilität den Aufwand einer Aktivierungsenergie, z.B. durch Einwirkung von Wärme, mechanischer Energie oder durch Einfluss eines Lösemittels.

Zudem ist allgemein bekannt, dass die verschiedenen Modifikationen einer Substanz monotrop oder enantiotrop vorliegen können. Im Fall der monotropen Polymorphie kann eine Kristallform bzw. Kristallmodifikation über den gesamten Temperaturbereich bis zum Schmelzpunkt die thermodynamisch stabile Phase darstellen, wohingegen bei enantiotropen Systemen ein Umwandlungspunkt existiert, bei dem sich das Stabilitätsverhältnis umkehrt.

Im Rahmen der vorliegenden Erfindung wurde gefunden, dass N-(Aminoiminomethyl)-2-aminoethansäure neben einer bereits bekannten, thermodynamisch stabilen Kristallmodifikation (im Folgenden auch Form A oder Kristallform A genannt) auch in einer thermodynamisch metastabilen Kristallmodifikation auftritt. Diese thermodynamisch metastabile Kristallform wird im Folgenden auch Form B oder Kristallform B genannt.

Diese thermodynamisch metastabile Kristallmodifikation (Form B) kann durch einfache Umkristallisation von N-(Aminoiminomethyl)-2-aminoethansäure aus Calciumchloridlösungen hergestellt werden. Es hat sich in den zugrundeliegenden Untersuchungen überraschender Weise auch gezeigt, dass diese bisher nicht bekannte, thermodynamisch metastabile Kristallmodifikation von N-(Aminoiminomethyl)-2-aminoethansäure auch mittels der direkten Synthese von N-(Aminoiminomethyl)-2-aminoethansäure in Calciumchloridlösungen hergestellt werden kann.

Somit ist gemäß einer ersten Ausführung der vorliegenden Erfindung ein Verfahren zur Herstellung einer thermodynamisch metastabilen Kristallmodifikation von N-(Aminoiminomethyl)-2-aminoethansäure Gegenstand der vorliegenden Erfindung, in dem Cyanamid und Glycin in einer wässrigen Calciumchlorid-haltigen Lösung umgesetzt werden und die Kristallmodifikation aus dem Reaktionsgemisch dieser Umsetzung kristallisiert wird.

Somit kann ein Verfahren bereitgestellt werden, indem das gewünschte Produkt direkt ohne eine nachfolgende Umkristallisation gewonnen wird.

Das Produkt der Umsetzung von Cyanamid und Glycin in einer wässrigen Calciumchlorid-haltigen Lösung ist eine thermodynamisch metastabile Kristallmodifikation von N-(Amino-imino-methyl)-2-aminoethansäure, die im Röntgen-Pulver-Diffraktogramm der Kristallmodifikation bei Verwendung von Cu-Kα₁-Strahlung die stärksten Reflexbanden bei 20 (2 Theta) = 20,2 ° und 23,3 ° und 23,8 ° und 25,3 ° bei einer Messgenauigkeit von +/- 0,2 ° zeigt.

Hierbei und im Folgenden bedeutet Cu-Kα₁-Strahlung eine Kupfer-K-alpha-1-Strahlung der Wellenlänge 1,5406 Å, wie sie üblicherweise in kristallographischen Untersuchungen herangezogen wird.

Das Produkt der Umsetzung von Cyanamid und Glycin in einer wässrigen Calciumchlorid-haltigen Lösung ist eine thermodynamisch metastabile Kristallmodifikation von N-(Amino-iminomethyl)-2-aminoethansäure, die in der orthorhombischen Raumgruppe P2₁2₁2₁ mit Z = 8, d.h. mit zwei kristallographisch unabhängigen Molekülen, kristallisiert und die insbesondere eine pseudo-tetragonale Packung aufweist. Die Elementarzelle weist bei 105 Kelvin die Gitterkonstanten a = 7,7685 Å, b = 7,7683 Å, c = 17,4261 Å bei einer Messgenauigkeit von +/- 0,001 Å auf. Die Einkristall-Messung erfolgte hierbei mit einer Mo-Kα-Strahlung der Wellenlänge 0,71073 Å bei 105 K (Kelvin). Gemäß der vorliegenden Erfindung bedeutet eine othorhombische Raumgruppe eine Raumgruppe, deren Elementarzelle drei rechte Winkel (rechter Winkel = 90 °) aufweist und die 3 Kristallachsen a, b und c unterschiedliche Längen aufweisen.

Gemäß einer Ausführung ist damit auch ein Verfahren zur Herstellung einer thermodynamisch metastabile Kristallmodifikation von N-(Aminoiminomethyl)-2-aminoethan-säure Gegenstand der vorliegenden Erfindung, die bevorzugt im Röntgen-Pulver-Diffraktogramm der Kristallmodifikation bei Verwendung von Cu-Kα₁-Strahlung die stärksten Reflexbanden bei 20 = 20,2 ° und 23,3 ° und 23,8 ° und 25,3 ° bei einer Messgenauigkeit von +/- 0,2 ° zeigt, und die weiter bevorzugt in der orthorhombischen Raumgruppe P2₁2₁2₁, insbesondere in der orthorhombischen, polaren Raumgruppe P2₁2₁2₁ mit Z = 8 vorliegt und die weiter bevorzugt eine pseudo-tetragonale Packung aufweist. Die Elementarzelle weist bei 105 Kelvin die Gitterkonstanten a = 7,7685 Å, b = 7,7683 Å, c = 17,4261 Å bei einer Messgenauigkeit von +/- 0,001 Å auf. Die Einkristall-Messung erfolgte hierbei mit einer Mo-Kα-Strahlung der Wellenlänge 0,71073 Å bei 105 K (Kelvin).

Diese neue Kristallform B bildet bei geeigneten Kristallisationsbedingungen polygonale oder kugelige, radialstrahlige Aggregate aus nadeligen Teilkristalliten, die einen rundlichen Habitus und eine weitgehend einheitliche Aggregatgröße aufweisen. Damit stellen sie eine optimale Handhabung als Feststoff sicher, indem sie ein staubfreies, gut rieselfähiges Produkt ohne Verbackungsneigung ermöglichen. Die Kristallmodifikation B kann als staubarm eingestuft werden, da der Anteil an Kristallen mit einer Korngröße von < 63 µm (Mesh-Größe) unter 10 % vorzugsweise unter 5 % liegt (vgl. Beispiele). Durch ihren Aufbau aus feinen, nadeligen Teilkristalliten stellt dieser Habitus der neuen Kristallform B von N-(Aminoiminomethyl)-2-aminoethansäure zudem eine höhere Lösungsgeschwindigkeit sicher. Zusätzlich und völlig unerwartet bietet N-(Aminoiminomethyl)-2-aminoethansäure der Kristallform B außerdem eine höhere absolute Löslichkeit in Wasser-haltigen Medien.

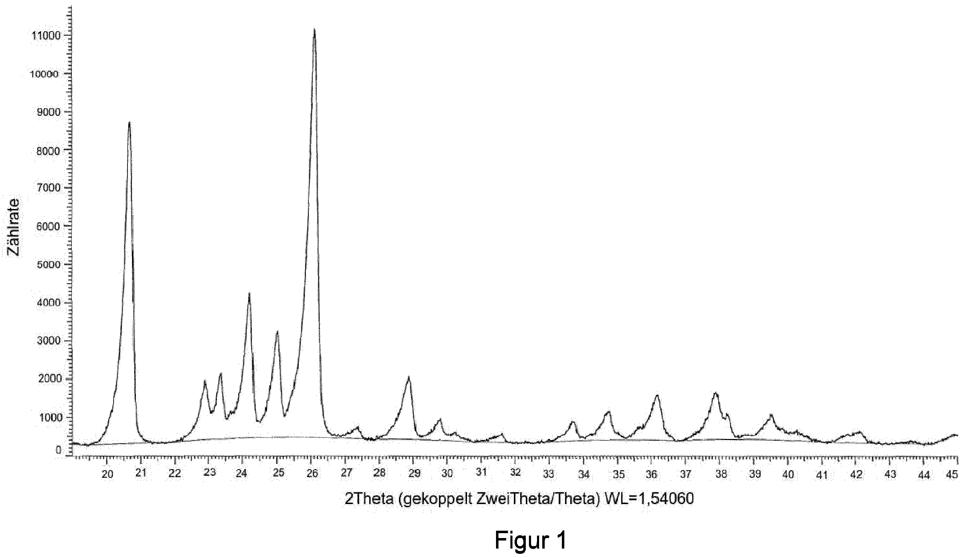

Wird N-(Aminoiminomethyl)-2-aminoethansäure nach einem der bekannten Verfahren, insbesondere aus wasserhaltigen Reaktionsmischungen, hergestellt, so fällt die Verbindung in der wohlbekannten Kristallform A an. Ein und dieselbe Kristallstruktur wurde von drei Autorengruppen beschrieben: von Sankarananda Guha, Acta Cryst. B29 (1973), 2163 bzw. von Par J. Berthou et. al., Acta Cryst B32 (1976), 1529 und von Wei Wang et. al, Tetrahedron Letters 56 (2015), 2684. In allen drei Arbeiten wird N-(Aminoiminomethyl)-2-aminoethansäure (hier Form A genannt) beschrieben als monokline Struktur der Raumgruppe P2₁/n mit Z = 4 und den angenäherten Gitterkonstanten a = 4,95 Å, b = 6,00 Å, c = 17,2 Å, β = 94,5 °, mit einem Zellvolumen von ca. 510 Å³, wobei bei Berthou et. al. die publizierte Raumgruppe P2₁/c über eine Koordinatentransformation zur Raumgruppe P2₁/n überführt wurde. Die experimentelle Kristalldichte von N-(Aminoiminomethyl)-2-aminoethansäure der Form A beträgt ca. 1,50 g/cm³. Das charakteristische Pulverdiffraktogramm von N-(Aminoiminomethyl)-2-aminoethansäure in Form A ist in Figur 1 gezeigt. Unter Verwendung von Cu-Kα₁-Strahlung (Kupfer-K-alpha-1-Strahlung) ist für Form A insbesondere die Bandenlage 20 (2Theta) = 20,6 ° und 26,0 ° charakteristisch. Das Pulverdiffraktogramm stimmt mit dem aus den publizierten Einkristallstrukturen berechneten Beugungsmuster überein.

Wird N-(Aminoiminomethyl)-2-aminoethansäure aus üblichen Lösemitteln, wie beispielsweise Wasser, Methanol, Ethanol, Isopropanol oder Mischungen aus Methanol, Ethanol, Ethandiol, oder Acetonitril mit Wasser, umkristallisiert oder darin hergestellt, so fällt N-(Aminoimino-methyl)-2-aminoethansäure ausschließlich in Kristallform A an, wie durch Versuche gezeigt werden konnte.

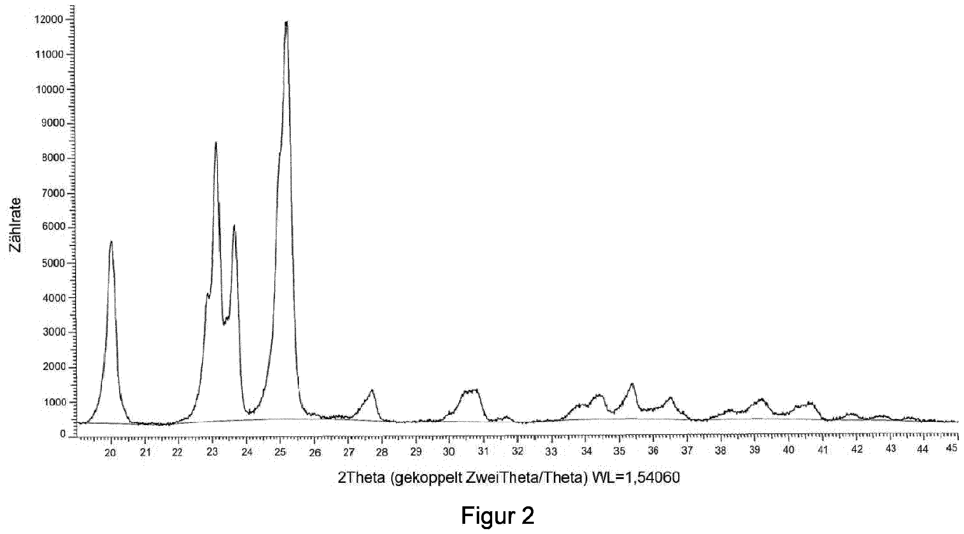

N-(Aminoiminomethyl)-2-aminoethansäure der Form B wird charakterisiert durch ihr Pulverdiffraktogramm mit Cu-Kα₁-Strahlung (siehe Figur 2), wobei die Banden bei 20 (2Theta) = 20,2 ° und 25,3 ° sowie ein schwächerer Doppelreflex bei 20 (2Theta) = 23,3 °/ 23,8 ° charakteristisch sind. Eine Einkristall-Röntgenstrukturanalyse ergab für N-(Aminoiminomethyl)-2-aminoethansäure der Form B die orthorhombische, polare Raumgruppe P2₁2₁2₁ mit zwei kristallographisch unabhängigen Molekülen, d.h. Z = 8. Die Packung der Moleküle weist eine pseudo-tetragonale Symmetrie auf. Die Elementarzelle weist bei 105 Kelvin die Gitterkonstanten a = 7,7685 Å, b = 7,7683 Å, c = 17,4261 Å bei einer Messgenauigkeit von +/- 0,001 Å auf. Die Einkristall-Messung erfolgte hierbei mit einer Mo-Kα-Strahlung der Wellenlänge 0,71073 Å. Das Elementarzellvolumen beträgt 1052 Å³ und die berechnete Röntgen-Kristalldichte 1,479 g/cm³ bei 105 Kelvin.

Die experimentelle Kristalldichte von N-(Aminoiminomethyl)-2-aminoethansäure der Form B beträgt 1,41 g/cm³ +/- 0,03 g/cm³ bei 20 °C. Somit liegt damit die experimentelle Kristalldichte der Form B deutlich unter derjenigen von Kristallform A, die 1,50 g/cm³ +/- 0,03 g/cm³ bei 20 °C beträgt. Dieser Unterschied in der Kristalldichte deutet auf eine thermodynamische Instabilität von Form B gegenüber Form A hin.

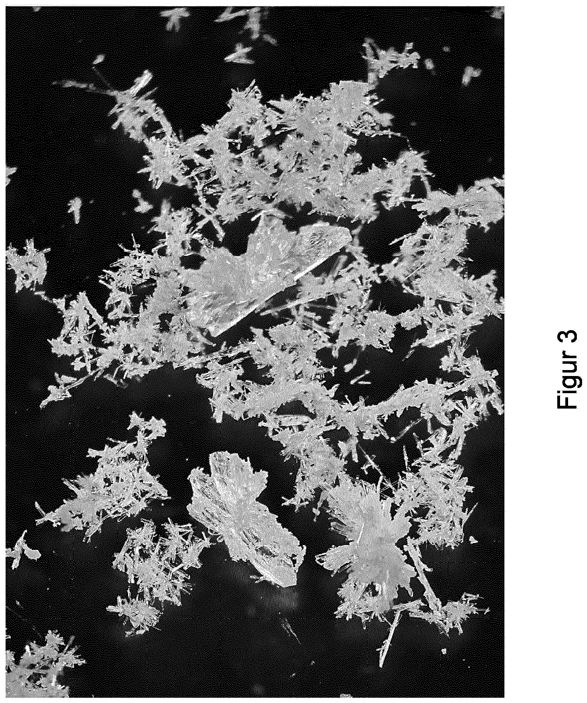

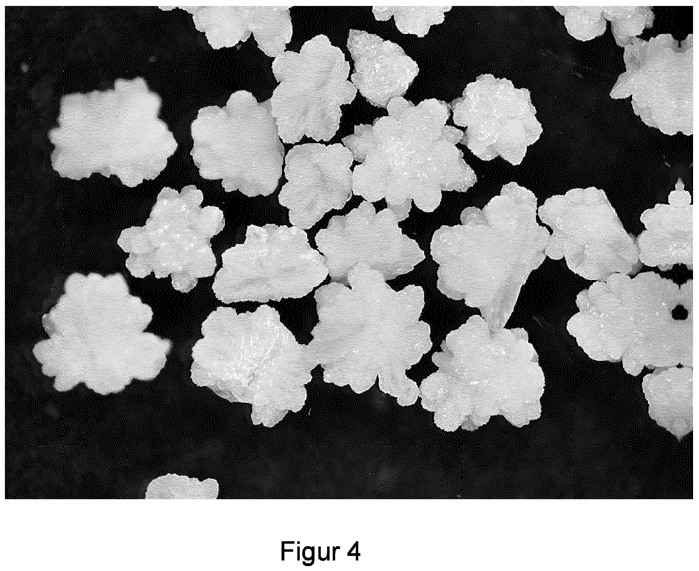

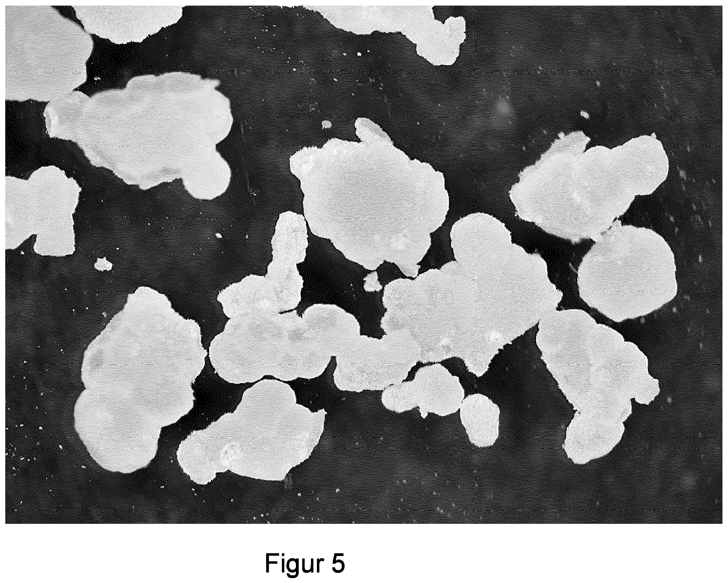

Kristallform B von N-(Aminoiminomethyl)-2-aminoethansäure liegt in Form kugeliger oder polygonaler, radialstrahliger Aggregate mit äußerem rundlichem Habitus vor. Die Einzelkristalle stellen feinste Nadeln dar, aus denen die kugeligen Aggregate aufgebaut sind. Dies hat den überraschenden Vorteil, dass mittels Form B eine physikalische Form von N-(Aminoiminomethyl)-2-aminoethansäure bereitgestellt werden kann, die kugelige oder polygonale, körnige, abriebfeste Aggregate umfasst, mit einer weitgehend einheitlichen Aggregatgröße, einer hervorragenden Rieselfähigkeit und weitgehender Staubfreiheit. Typische Kristallaggregate von N-(Aminoiminomethyl)-2-aminoethansäure der Form B sind in Figur 4 und Figur 5 gezeigt. Zum Vergleich wird übliche, dem Stand der Technik entsprechende N-(Aminoiminomethyl)-2-aminoethansäure der Form A, die den Habitus verfilzter, feiner Kristallnadeln aufweist, in Figur 3 gezeigt. N-(Aminoiminomethyl)-2-aminoethansäure Form A und Form B unterscheiden sich zudem im Infrarotspektrum. Charakteristisch für Form A sind stärkere Banden bei 1005,9, 940,3 und 816,8 cm⁻¹, charakteristisch für Form B sind stärkere Banden bei 1148,0, 997,7 und eine nur schwache Bande bei 815 cm⁻¹.

Die beiden Kristallformen zeigen unterschiedliche Schmelz- bzw. Zersetzungspunkte:
N-(Aminoiminomethyl)-2-aminoethansäure Form A: DSC onset 280,5 °C, peak 286,3 °C, Schmelzwärme 887 +/- 1 J/g.
N-(Aminoiminomethyl)-2-aminoethansäure Form B: DSC onset 272,5 °C, peak 280,4 °C, Schmelzwärme 860 +/- 1 J/g.

Diese Daten zeigen eindrucksvoll, dass N-(Aminoiminomethyl)-2-aminoethansäure Form B eine thermodynamisch metastabile Kristallmodifikation ist, die im Vergleich zur Form A die thermodynamisch instabilere Form darstellt, wobei der Energieunterschied zwischen beiden Formen ca. 27 J/g beträgt und wobei der Anfangspunkt der Schmelzbereiche (onset) einen Unterschied von 8 K aufweist.

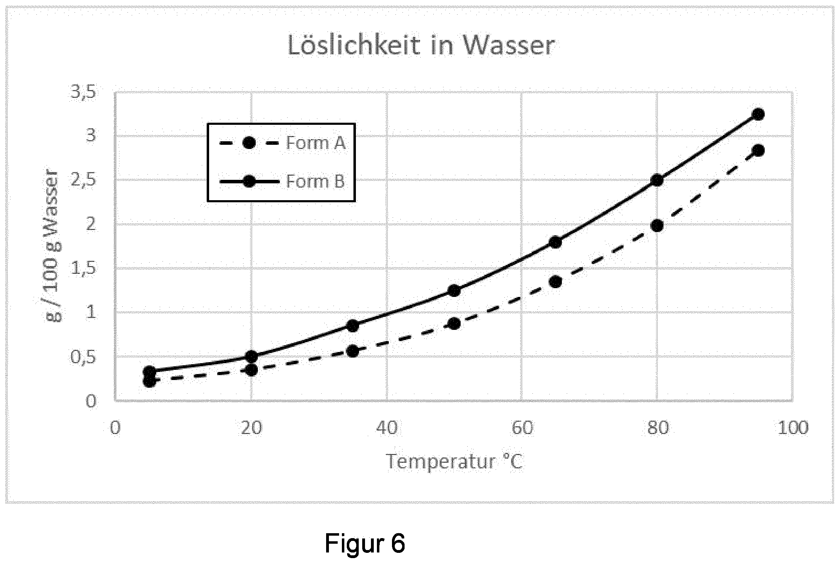

In weitergehenden Untersuchungen hat sich gezeigt, dass N-(Aminoiminomethyl)-2-aminoethansäure Form B eine um ca. 20 % höhere Wasserlöslichkeit als N-(Aminoiminomethyl)-2-aminoethansäure Form A aufweist, und dass dieser Sachverhalt im Temperaturbereich zwischen 5 und 95 °C zutrifft (vgl. Figur 6). Dieser Effekt ist vollkommen unvorhersehbar.

Es wurde gefunden, dass die metastabile Kristallmodifikation Form B als Feststoff bis zu ihrem Schmelzpunkt stabil vorliegt. Eine Feststoffumwandlung von Form B in die Form A oder eine reversible Feststoffumwandlung Form A / Form B kann nicht beobachtet werden. Somit liegt mit Form B ein Beispiel für eine monotrope Polymorphie vor.

Zusammenfassend ist hier hervorzuheben, dass N-(Aminoiminomethyl)-2-aminoethansäure in der Kristallmodifikation B, insbesondere hergestellt durch Kristallisation von N-(Amino-iminomethyl)-2-aminoethansäure aus einer CaCl₂ enthaltenden, bevorzugt wässrigen oder wasserhaltigen CaCl₂-Lösung, überraschenderweise vorteilhafte und gewöhnlich entgegengesetzte Eigenschaften, wie z.B. ein grobes, rieselfähiges Korn und zugleich eine hohe Lösungsgeschwindigkeit, die Bildung von Kristallaggregaten ohne Zusatz eines Bindemittels, kombiniert, und eine erhöhte absolute Löslichkeit bei gegebener Temperatur, trotz identischer chemischer Zusammensetzung bereitstellt.

Diese neue Kristallmodifikation ist aufgrund ihrer hervorragenden Eigenschaften geeignet, als Futtermittelzusatz für Tiere eingesetzt zu werden. Somit ist auch ein Futtermittelzusatz umfassend die hierin beschriebene thermodynamisch metastabile Kristallmodifikation von N-(Aminoiminomethyl)-2-amino-ethansäure Gegenstand der vorliegenden Erfindung.

Insbesondere ist somit auch ein Futtermittelzusatz umfassend eine thermodynamisch metastabile Kristallmodifikation von N-(Aminoiminomethyl)-2-aminoethansäure Gegenstand der vorliegenden Beschreibung dessen Röntgen-Pulver-Diffraktogramm bei Verwendung von Cu-Kα₁-Strahlung die stärksten Reflexbanden bei 2Θ = 20,2 ° und 23,3 ° und 23,8 ° und 25,3 ° bei einer Messgenauigkeit von +/-0,2 ° zeigt.

Ganz besonders bevorzugt genannt ist ein Futtermittelzusatz umfassend eine thermodynamisch metastabile Kristallmodifikation von N-(Aminoiminomethyl)-2-aminoethansäure, der keine Bindemittel umfasst oder der frei von Bindemitteln ist, die üblicherweise zur Granulation eingesetzt werden.

Derartige Futtermittelzusätze können als Premixe formuliert werden. Somit ist zudem auch die Verwendung der hierin beschriebenen thermodynamisch metastabilen Kristallmodifikation von N-(Aminoiminomethyl)-2-amino-ethansäure zur Herstellung eines Futtermittelzusatzes Gegenstand der Erfindung. Der Futtermittelzusatz ist insbesondere zur Fütterung von Geflügel geeignet.

Es hat sich in den zugrundeliegenden Untersuchungen überraschender Weise auch gezeigt, dass diese bisher nicht bekannte, thermodynamisch metastabile Kristallmodifikation von N-(Aminoiminomethyl)-2-aminoethansäure mittels der direkten Synthese von N-(Amino-iminomethyl)-2-aminoethansäure in Calciumchloridlösungen hergestellt werden kann.

Somit ist ein Verfahren zur Herstellung einer thermodynamisch metastabilen Kristallmodifikation von N-(Aminoimino-methyl)-2-aminoethansäure Gegenstand der vorliegenden Erfindung, in dem Cyanamid und Glycin in einer wässrigen Calciumchlorid-haltigen Lösung umgesetzt werden und die Kristallmodifikation aus dem Reaktionsgemisch dieser Umsetzung kristallisiert wird.

Bevorzugt kann das erfindungsgemäße Verfahren durchgeführt werden, indem die wässrige Lösung mindesten 5 bis maximal 50 Gew-% Calciumchlorid (bezogen auf das Reaktionsgemisch), weiter bevorzugt 5 bis 40 Gew-%, besonders bevorzugt 10 bis 40 Gew-% Calciumchlorid (bezogen auf das Reaktionsgemisch) enthält.

Weiterhin bevorzugt kann das Calciumchlorid (bezogen auf das wasserfreie Salz) in einer Menge von mindestens 5 Gew.-% und höchstens 50 Gew.-% (bezogen auf das Gesamtgewicht der Lösung) eingesetzt werden. Bevorzugt kann das Calciumchlorid in einer Menge von mindestens 7 Gew.-%, weiter bevorzugt mindestens 10 Gew.-%, besonders bevorzugt mindestens 15 Gew.-% und ganz besonders bevorzugt mindestens 20 Gew.-%, wobei weiterhin bevorzugt höchstens 50 Gew.-% (jeweils bezogen auf das Gesamtgewicht der Lösung) eingesetzt werden. Gleichzeitig kann das Calciumchlorid in einer Menge von höchstens 50 Gew.-%, weiter bevorzugt von höchstens 45 Gew.-% und ganz besonders bevorzugt höchstens 40 Gew.-% (jeweils bezogen auf das Gesamtgewicht der Lösung) eingesetzt werden.

Somit ist auch ein Verfahren zur Herstellung einer thermodynamisch metastabilen Kristallmodifikation von N-(Aminoiminomethyl)-2-aminoethansäure Gegenstand der vorliegenden Erfindung, in dem Cyanamid und Glycin in einer wässrigen Calciumchlorid-haltigen Lösung umgesetzt werden, wobei die wässrige Lösung 5 bis 50 Gew-% Calciumchlorid (bezogen auf das Reaktionsgemisch) enthält und die Kristallmodifikation aus dem Reaktionsgemisch dieser Umsetzung kristallisiert wird. Die wässrige Calciumchlorid-haltigen Lösung kann als Lösemittel ausschließlich Wasser enthalten. Es können jedoch auch weitere Lösemittel, insbesondere organische Lösemittel, insbesondere organische Lösemittel aus der Gruppe der mit Wasser mischbaren organischen Lösemittel, insbesondere Alkohole, Tetrahydrofuran oder Aceton eingesetzt werden. Als besonders geeignet haben sich mit Wasser mischbare Alkohole, insbesondere aus der Gruppe Methanol, Ethanol, Propanol, Isopropanol und Butanol oder Mischungen dieser Alkohole gezeigt.

Bevorzugt ist ein Verfahren, in dem die wässrige Calciumchlorid-Lösung ausschließlich Wasser enthält.

Weiterhin bevorzugt ist ein Verfahren, indem das Glycin in der wässrigen Calciumchlorid-haltigen Lösung vorgelegt wird und das Cyanamid zugegeben wird. Die Zugabe von Cyanamid kann dabei als Feststoff oder in Form einer Cyanamid-haltigen Lösung, insbesondere einer wässrigen Cyanamid-haltigen Lösung, erfolgen.

Somit ist auch ein Verfahren Gegenstand der Erfindung, in dem das Glycin in der wässrigen Calciumchlorid-haltigen Lösung vorgelegt wird und das Cyanamid als Feststoff oder in Form einer wässrigen Lösung in die Calciumchlorid-haltige Lösung oder das Reaktionsgemisch zugegeben wird. Bevorzugt wird das Cyanamid oder als wässrige Cyanamid-Lösung in die Calciumchlorid-haltige Lösung oder das Reaktionsgemisch zugegeben.

Besonders bevorzugt ist somit auch ein Verfahren Gegenstand der Erfindung, in dem die wässrige Lösung und/oder das Reaktionsgemisch neben Wasser kein weiteres Lösungsmittel umfasst. Derartig geführte Verfahren zeichnen sich durch den entscheidenden Vorteil aus, dass die zurückbleibenden Reaktionsmedien aufgrund einer Belastung von verschiedenen Lösemitteln bzw. deren Trennung von Wasser keiner besonderen Aufarbeitung unterliegen müssen.

Empfehlenswert ist es auch, die Reaktanden der Umsetzung, nämlich Cyanamid und Glycin, bei einer Reaktionstemperaturen im Bereich von 20 bis 100 °C, vorzugsweise im Bereich von 60 bis 100 °C, zur Reaktion zu bringen und somit umzusetzen. Dies kann bei Normaldruck, unter Vakuum oder auch unter Druck stattfinden. Bevorzugt kann die Umsetzung bei Normaldruck in einem Temperaturbereich von 20 bis 100 °C erfolgen.

Allerdings empfiehlt es sich auch, die Umsetzung bei einem pH-Wert im Bereich von 7,0 bis 10,0, vorzugsweise bei einem pH-Wert im Bereich von 8,0 bis 10,0 durchzuführen. Der pH-Wert wird mit einer geeigneten Base eingestellt, bei denen es sich sowohl um organische als auch anorganische Basen handeln kann. Bevorzugt können hierbei Natriumhydroxid, Kaliumhydroxid, Calciumhydroxid, Ammoniak oder deren wässrige Lösungen eingesetzt werden. Besonders bevorzugt können Natriumhydroxid, Calciumhydroxid und deren wässrige Lösungen eingesetzt werden.

Die Reaktion läuft bei diesen Bedingungen ohne Komplikationen ab, wobei sich das Produkt nach und nach in dem Reaktionsgemisch bildet. Nach beendeter Zugabe von Cyanamid kann das Reaktionsgemisch noch einige Zeit nachreagieren. Sofern der Sättigungspunkt, d.h. die maximale Konzentration bei der Reaktionstemperatur erreicht ist, beginnt die Kristallisation. Gemäß dem erfindungsgemäßen Verfahren erfolgt die Kristallkeimbildung und Kristallisation vorzugsweise in Form B, wobei die Anwesenheit von Calciumchlorid als erfindungswesentlich anzusehen ist. Die Kristallisation des gewünschten Produktes kann bei einer Temperatur im Bereich von -40 bis 100 °C erfolgen.

Überraschenderweise wurde zudem gefunden, dass die Anwesenheit von Calciumchlorid in dem Reaktionsgemisch die Löslichkeit von N-(Aminoiminomethyl)-2-aminoethansäure sehr deutlich erhöht. Bevorzugt kann das Verfahren somit durchgeführt werden, indem die N-(Aminoiminomethyl)-2-aminoethansäure in einem Temperaturbereich von -40 bis 100 °C, insbesondere in einem Temperaturbereich von -40 bis 70 °C, weiter bevorzugt in einem Temperaturbereich von -40 bis 50 °C, weiter bevorzugt in einem Temperaturbereich von -40 bis 40 °C kristallisiert wird.

Besonders bevorzugt ist jedoch ein Verfahren in dem die Kristallisation kontrolliert erfolgt. Hierbei wird das Reaktionsgemisch in konstant gehaltenen Zeitabschnitten definierten Temperaturunterschieden ausgesetzt. Hierdurch kann eine besonders gleichförmige Kristallbildung erreicht werden.

Besonders bevorzugt ist somit ein Verfahren, indem N-(Aminoiminomethyl)-2-aminoethansäure mit einer Abkühlrate im Bereich von 0,01 bis 5 K/min, weiter bevorzugt im Bereich von 0,1 bis 5 K/min, weiter bevorzugt im Bereich von 0,5 bis 5 K/min, in einem Temperaturbereich von -40 bis 100 °C kristallisiert wird.

In jedem Fall werden vorzugsweise gewöhnliche Rührreaktoren eingesetzt. Der Einsatz aufwendiger verfahrenstechnischer Apparate ist nicht erforderlich.

Nach vollständiger Kristallisation der gewünschten N-(Aminoiminomethyl)-2-aminoethansäure Form B wird das auskristallisierte Produkt vorzugsweise durch Filtration, z.B. mittels Zentrifuge, Druckfilternutsche, Bandfilter oder Filterpresse abfiltriert. Zur Entfernung überschüssigen Calciumchlorids wird vorzugsweise mit dem obengenannten Lösemittel oder Lösemittelgemisch nachgewaschen. Vorzugsweise wird mit Wasser gewaschen, wobei die Temperatur des Waschwassers bevorzugt 0 bis 50 °C beträgt.

Selbstverständlich ist es zur Verbesserung der Wirtschaftlichkeit des Verfahrens möglich, die aus der Abtrennung der N-(Aminoiminomethyl)-2-aminoethansäure der Kristallform B erhaltene Mutterlauge in den Prozess zurückzuführen, ggf. unter Einstellung der Konzentration von Calciumchlorid, z.B. durch Eindampfung. Nach Trocknung, vorzugsweise im Temperaturbereich 40 bis 100 °C, liefert das erfindungsgemäße Verfahren ein trockenes, rieselfähiges, körniges Produkt bestehend aus radialstrahligen, polygonalen oder rundlichen Aggregaten. Die Kristallaggregate haben eine äußere Abmessung von 150 bis 3000 µm, vorzugsweise 300 bis 1500 µm und einen Staubanteil (d.h. Partikelanteil kleiner 63 µm) von weniger als 5 Gew.-%.

Die so hergestellte N-(Aminoiminomethyl)-2-aminoethansäure Form B hat eine hohe Reinheit, typischerweise > 99,0 %, ist gut handhabbar und zeigt kaum mechanischen Abrieb. Aufgrund dieser Eigenschaften ist die Kristallform B von N-(Aminoiminomethyl)-2-aminoethansäure besonders geeignet für die obengenannten Einsatzzwecke, insbesondere als Zusatzstoff zur Ernährung bzw. als pharmazeutischer Wirkstoff.

Die metastabile Kristallmodifikation von N-(Aminoiminomethyl)-2-aminoethansäure liegt bevorzugt in reiner Form vor. Vorzugsweise weisen mindestens 50 Gew.-%, mehr bevorzugt mindestens 75 Gew.-%, noch mehr bevorzugt mindestens 90 Gew.-%, insbesondere mindestens 95 Gew.-% und am meisten bevorzugt mindestens 99 Gew.-% der N-(Aminoiminomethyl)-2-aminoethansäure in einer Zusammensetzung eine Kristallmodifikation im Röntgen-Pulver-Diffraktogramm bei Verwendung von Cu-Kα₁-Strahlung mit stärksten Reflexbanden bei 20 = 20,2 ° und 23,3 ° und 23,8 ° und 25,3 ° bei einer Messgenauigkeit von +/- 0,2 ° auf.

Somit ist gemäß einem weiterführenden Gedanken auch die Verwendung der thermodynamisch metastabilen Kristallmodifikation von N-(Aminoiminomethyl)-2-aminoethansäure zur Herstellung eines pharmazeutischen Wirkstoffs, zur Herstellung eines Nahrungsergänzungsmittels oder zur Herstellung eines Futtermittelzusatzes Gegenstand der vorliegenden Erfindung.

Die nachfolgenden Beispiele sollen das Wesen der Erfindung näher erläutern.

In den Abbildungen wird gezeigt:
- Figur 1:: Röntgen-Pulver-Diffraktogramm von N-(Aminoiminomethyl)-2-aminoethansäure der Form A aus Beispiel 1
- Figur 2:: Röntgen-Pulver-Diffraktogramm von N-(Aminoiminomethyl)-2-aminoethansäure der Form B aus Beispiel 2
- Figur 3:: Mikrophotographie von N-(Aminoiminomethyl)-2-aminoethansäure der Form A, hergestellt nach Beispiel 1 (Bildbreite 8 mm)
- Figur 4:: Mikrophotographie von polygonalen Aggregaten von N-(Aminoiminomethyl)-2-aminoethansäure der Form B, hergestellt durch Umkristallisation aus einer 30 %-igen wässrigen Calciumchloridlösung gemäß Beispiel 2 (Bildbreite 8 mm)
- Figur 5:: Mikrophotographie von kugeligen Aggregaten von N-(Aminoiminomethyl)-2-aminoethansäure der Form B, hergestellt durch Umkristallisation aus einer 15 %-igen wässrigen Calciumchloridlösung gemäß Beispiel 3 (Bildbreite 8 mm)
- Figur 6:: Löslichkeitskurve von N-(Aminoiminomethyl)-2-aminoethansäure der Form A bzw. Form B in Wasser
- Figur 7:: Abbildung der beiden kristallographisch unabhängigen Moleküle N-(Aminoiminomethyl)-2-aminoethansäure aus der Einkristall-Röntgenstrukturanalyse
- Figur 8:: Abbildung der Packung der Moleküle von N-(Aminoiminomethyl)-2-aminoethansäure im Kristallverband. Die Blickrichtung ist entlang der a-Achse. Deutlich sind voneinander unabhängige, senkrecht zueinander angeordnete, über H-Brücken gebundene Molekülketten parallel der a- und der b-Achse zu sehen. Diese Ketten sind entlang der c-Achse gestapelt.

### Beispiele

### Röntgen-Pulver-diffraktometrische Messung

Im Umfang der vorliegenden Beispiele wurden Röntgen-Pulver-diffraktometrische Messungen unter Verwendung eines Pulver-Diffraktometers Bruker D2 Phaser mit Theta/2Theta-Geometrie, einem LYNXEYE-Detektor, Cu-Kα₁-Strahlung der Wellenlänge 1,5406 Å mit einer Beschleunigungsspannung von 30 kV und einem Anodenstrom von 10 mA, einem Nickelfilter und einer Schrittweite von 0,02 ° durchgeführt. Die zur Untersuchung stehenden Proben wurden im Achatmörser vermahlen und gemäß Herstellerangaben auf den Probenteller gedrückt und die Oberfläche geglättet.

### Einkristall-Röntgenstrukturanalyse

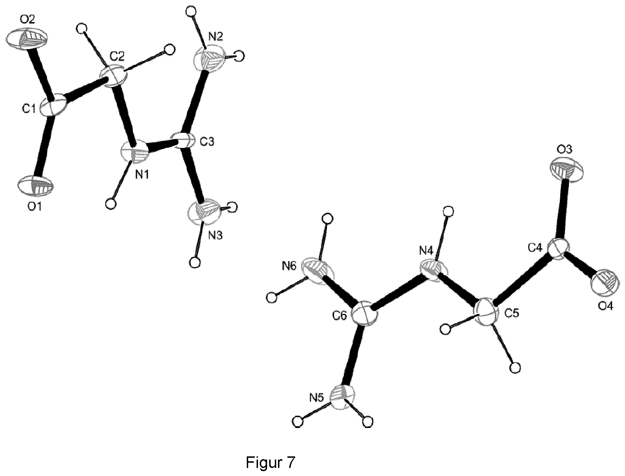

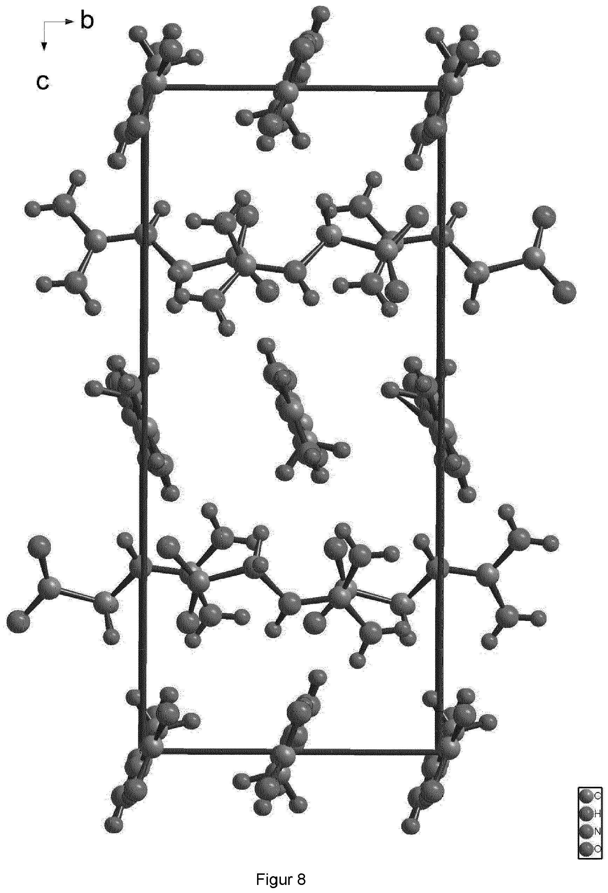

Ein geeigneter Kristall wurde durch Verdunsten einer wässrigen Lösung von N-(Aminoiminomethyl)-2-aminoethansäure in Gegenwart von Calciumchlorid hergestellt. Die Einkristallmessung erfolgte bei 105 Kelvin an einem Kristall der Dimension 0,02 * 0,02 * 0,09 mm unter Verwendung monochromatischer Mo-Kα (Molybdän-K-alpha)-Strahlung der Wellenlänge 0,71073 Å unter Einsatz eines Zweikreis-Diffraktometers Bruker D8 Venture TXS. Die Verfeinerung der Röntgenkristalldaten unter Verwendung von 2072 unabhängigen Reflexen erfolgte nach der Methode der kleinsten Fehlerquadrate bis zu einem R-Wert (F_{obs}) von 0,0381. Die Position der NH- und OH-Wasserstoffatome wurde verfeinert, die der CH-Wasserstoffatome an der berechneten Position fixiert. Das Ergebnis der Röntgen-Einkristallstrukturanalyse ist in Figur 7 und 8 veranschaulicht. Ein aus der Einkristallstrukturanalyse rückgerechnetes Pulverdiffraktogramm stimmte exakt mit dem gemessenen Pulverdiffraktogramm gemäß Figur 2 überein.

### Beispiel 1 (Vergleich) - Umkristallisation von N-(Aminoiminomethyl)-2-aminoethansäure aus Wasser

400 g Wasser wurden bei 80 °C vorgelegt und löffelweise insgesamt 11,66 g N-(Aminoimino-methyl)-2-aminoethansäure mit einem Gehalt von 99,0 %, vorliegend in Kristallform A, darin gelöst, wobei mit der letzten Portion die Löslichkeitsgrenze überschritten wurde. Dann wurde bei 80 °C abfiltriert, das Filtrat mit weiteren 100 g Wasser versetzt und auf 80 °C erhitzt. Es bildete sich eine knapp gesättigte, klare Lösung. Durch langsame Abkühlung auf 20 °C innerhalb von 4 Stunden wurde N-(Aminoiminomethyl)-2-aminoethansäure kristallisiert. Die ausgefallenen Kristalle wurden abfiltriert und bei 60 °C im Vakuum getrocknet. Es wurden 6,51 g N-(Aminoiminomethyl)-2-aminoethansäure mit einem Gehalt von 99,1 % erhalten.

Das erhaltene Produkt liegt in Form fein-nadeliger Kristalle vor. Die fein-nadeligen Kristalle wurden mikroskopisch untersucht (siehe Figur 3). Eine Röntgen-Pulver-diffraktometrische Messung ergab das mit Figur 1 gezeigte Pulverdiffraktogramm, welches die altbekannte Kristallform A anzeigt.

### Beispiel 2 (erfindungsgemäß) - Umkristallisation von N-(Aminoiminomethyl)-2-aminoethansäure aus einer 30 %-igen Calciumchloridlösung

Aus 150 g wasserfreiem Calciumchlorid und 350 g Wasser wurde eine 30 %-ige Lösung hergestellt. In 400 g dieser Lösung wurde bei 80 °C löffelweise N-(Aminoiminomethyl)-2-aminoethansäure derselben Zusammensetzung wie in Beispiel 1 (d.h. 99,0 % Gehalt, Kristallform A) zugesetzt. Erst bei einer zugegebenen Menge von 74,28 g war die Löslichkeitsgrenze überschritten. Der geringe Feststoffanteil wurde bei 80 °C abfiltriert, nicht gewaschen, das Filtrat mit den restlichen 100 g der 30 %-igen Lösung von Calciumchlorid versetzt und bei 80 °C für 1 Stunde gerührt. Es wurde eine klare, farblose Lösung erhalten. Durch langsame Abkühlung auf 20 °C innerhalb von 4 Stunden wurde N-(Aminoiminomethyl)-2-aminoethansäure kristallisiert. Die ausgefallenen Kristallaggregate wurden abfiltriert, 3 mal mit Wasser von 20 °C gewaschen und bei 60 °C getrocknet. Es wurden 46,42 g N-(Aminoiminomethyl)-2-aminoethansäure mit einem Gehalt von 99,2 % erhalten. Die erhaltene Menge ist somit über 7 mal größer als in Beispiel 1, was auf die durch Calciumchlorid stark erhöhte Löslichkeit von N-(Aminoiminomethyl)-2-aminoethansäure zurückzuführen ist.

Ein analog aufgenommenes Pulverdiffraktogramm (siehe Figur 2) zeigte die bislang unbekannte Kristallform B an. Die polygonalen, rundlichen Kristallaggregate wurden mikroskopisch untersucht (siehe Figur 4).

### Beispiel 3 (erfindungsgemäß) - Umkristallisation von N-(Aminoiminomethyl)-2-aminoethansäure aus einer 15 %-igen Calciumchloridlösung

Beispiel 2 wurde analog wiederholt mit 500 g einer 15 %-igen Calciumchloridlösung, hergestellt aus 75 g wasserfreiem Calciumchlorid und 425 g Wasser. In 400 g dieses Lösemittelgemisches wurde bei 80 °C die Sättigungsgrenze mit 42,7 g N-(Aminoiminomethyl)-2-aminoethansäure erreicht. Nach Zugabe der restlichen 100 g Lösemittel, Kristallisation der anfangs klaren Lösung, Filtration, Waschen und Trocknen wurden 27,2 g N-(Aminoiminomethyl)-2-aminoethansäure mit einem Gehalt von 99,2 % erhalten.

Das Pulverdiffraktogramm der kugeligen Kristallaggregate zeigte die alleinige Anwesenheit von Form B an. Die kugeligen, radialstrahligen Aggregate wurden mikroskopisch untersucht (siehe Figur 5).

### Beispiel 3a (erfindungsgemäß) - Umkristallisation von N-(Aminoiminomethyl)-2-aminoethansäure aus einer 10 %-igen Calciumchloridlösung

Beispiel 2 wurde analog wiederholt mit 500 g einer 10 %-igen Calciumchloridlösung, hergestellt aus 50 g wasserfreiem Calciumchlorid und 450 g Wasser. In 400 g dieses Lösemittelgemisches wurde bei 80 °C die Sättigungsgrenze mit 29,4 g N-(Aminoiminomethyl)-2-aminoethansäure erreicht. Nach Zugabe der restlichen 100 g Lösemittel, Kristallisation der anfangs klaren Lösung, Filtration, Waschen und Trocknen wurden 23,5 g N-(Aminoiminomethyl)-2-aminoethansäure mit einem Gehalt von 99,3 % erhalten.

Das Pulverdiffraktogramm zeigte die Anwesenheit einer Mischung von Kristallform A und Kristallform B an. Das Verhältnis beider Polymorphe betrug ca. 1:1.

### Beispiel 3b (Vergleich) - Umkristallisation von N-(Aminoiminomethyl)-2-aminoethansäure aus einer 1 %-igen Calciumchloridlösung

Beispiel 2 wurde analog wiederholt mit 500 g einer 1 %-igen Calciumchloridlösung, hergestellt aus 5 g wasserfreiem Calciumchlorid und 495 g Wasser. In 400 g dieses Lösemittelgemisches wurde bei 80 °C die Sättigungsgrenze mit 13,4 g N-(Aminoiminomethyl)-2-aminoethansäure erreicht. Nach Zugabe der restlichen 100 g Lösemittel, Kristallisation der anfangs klaren Lösung, Filtration, Waschen und Trocknen wurden 11,0 g N-(Aminoiminomethyl)-2-aminoethansäure mit einem Gehalt von 99,4 % erhalten.

Das Pulverdiffraktogramm der fein-nadeligenKristallaggregate zeigte die alleinige Anwesenheit von Form A an.

Abhängig von der Calciumchloridkonzentration lässt sich somit das Entstehen von Form A oder Form B beeinflussen. Die Löslichkeit (d.h. Sättigungsgrenze) von N-(Aminoiminomethyl)-2-aminoethansäure steigt mit der Calciumchloridkonzentration stark an.

### Beispiel 4 (Vergleich) - Umkristallisation von N-(Aminoiminomethyl)-2-aminoethansäure aus einer 50 %-igen Lösung von Magnesiumchlorid-Hexahydrat

Beispiel 2 wurde analog wiederholt mit 500 g einer Lösung hergestellt aus 250 g Magnesiumchlorid-Hexahydrat und 250 g Wasser. In 400 g des Lösemittelgemisches wurde bei 80 °C die Sättigungsgrenze mit 76,6 g N-(Aminoiminomethyl)-2-aminoethansäure erreicht. Nach Zugabe der restlichen 100 g Lösemittel, Kristallisation, Filtration, Waschen und Trocknen wurden 49,1 g N-(Aminoiminomethyl)-2-aminoethansäure mit einem Gehalt von 99,1 % erhalten.

Das Pulverdiffraktogramm der erhaltenen nadeligen Kristalle zeigte die alleinige Anwesenheit von Form A an. Das zu CaCl₂ sehr ähnliche MgCl₂ bewirkt also nicht die Kristallisation von N-(Aminoiminomethyl)-2-aminoethansäure in Form B, obwohl die Löslichkeit von N-(Aminoiminomethyl)-2-aminoethansäure durch die Anwesenheit des Salzes ähnlich stark erhöht wird.

### Beispiel 5 (Vergleich) - Synthese von N-(Aminoiminomethyl)-2-aminoethansäure aus Glycin und Cyanamid in wässriger Lösung

112,6 g (1,5 mol) Glycin wurden in 300 g Wasser gelöst. Die Lösung wurde mit 21,6 g (0,27 mol) einer 50 %-igen Natronlauge versetzt wobei sich ein pH-Wert von 8,4 ergab. Bei 80 °C wurde im Verlauf von 4 Stunden eine Lösung von 42,04 g (1,0 mol) Cyanamid gelöst in 42 g Wasser zudosiert. Die Nachreaktion erfolgte für eine weitere Stunde bei 80 °C. Die erhaltene Suspension wurde auf 20 °C abgekühlt, abfiltriert, mit Wasser gewaschen und bei 60 °C getrocknet. Es wurden 100,6 g N-(Aminoiminomethyl)-2-aminoethansäure mit einem Gehalt von 99,1 % erhalten. Die Ausbeute betrug 85,9 %.

Ein Pulverdiffraktogramm der erhaltenen fein-nadeligen Kristalle zeigte die alleinige Anwesenheit von Form A an.

Beispiel 6 (erfindungsgemäß) - Synthese von N-(Aminoiminomethyl)-2-aminoethansäure aus Glycin und Cyanamid in einer 33 %-igen Calciumchloridlösung

Aus 100 g wasserfreiem Calciumchlorid und 200 g Wasser wurde eine Lösung hergestellt. Darin wurden 112,6 g (1,5 mol) Glycin gelöst und mit 21,6 g (0,27 mol) einer 50 %-igen Natronlauge ein pH-Wert von 8,4 eingestellt. Bei 80 °C wurde im Verlauf von 4 Stunden eine Lösung von 42,04 g (1,0 mol) Cyanamid gelöst in 42 g Wasser zudosiert. Die Nachreaktion erfolgte für eine weitere Stunde bei 80 °C. Die erhaltene Suspension wurde auf 20 °C abgekühlt, abfiltriert, mit Wasser gewaschen und bei 60 °C getrocknet. Es wurden 99,3 g N-(Aminoiminomethyl)-2-aminoethansäure mit einem Gehalt von 99,2 % erhalten. Die Ausbeute betrug 84,8 %.

Ein Pulverdiffraktogramm der erhaltenen rundlichen Kristallaggregate aus radialstrahligen Einzelkristallen zeigte die alleinige Anwesenheit von Form B an. Beispiel 6a (erfindungsgemäß) - Synthese von N-(Aminoiminomethyl)-2-aminoethansäure aus Glycin und Cyanamid in einer 15 %-igen Calciumchloridlösung

Aus 45 g wasserfreiem Calciumchlorid und 255 g Wasser wurde eine Lösung hergestellt. Darin wurden 112,6 g (1,5 mol) Glycin gelöst und mit 21,5 g (0,27 mol) einer 50 %-igen Natronlauge ein pH-Wert von 8,4 eingestellt. Bei 80 °C wurde im Verlauf von 4 Stunden eine Lösung von 42,04 g (1,0 mol) Cyanamid gelöst in 42 g Wasser zudosiert. Die Nachreaktion erfolgte für eine weitere Stunde bei 80 °C. Die erhaltene Suspension wurde auf 20 °C abgekühlt, abfiltriert, mit Wasser gewaschen und bei 60 °C getrocknet. Es wurden 99,6 g N-(Aminoiminomethyl)-2-aminoethansäure mit einem Gehalt von 99,3 % erhalten. Die Ausbeute betrug 84,5 %.

Ein Pulverdiffraktogramm der erhaltenen Kristalle zeigte, dass eine Mischung von Form A und Form B vorlag, wobei Form B den weitaus größeren Anteil ausmachte.

### Beispiel 6b (Vergleich) - Synthese von N-(Aminoiminomethyl)-2-aminoethansäure aus Glycin und Cyanamid in einer 1 %-igen Calciumchloridlösung

Aus 3 g wasserfreiem Calciumchlorid und 297 g Wasser wurde eine Lösung hergestellt. Darin wurden 112,6 g (1,5 mol) Glycin gelöst und mit 21,4 g (0,27 mol) einer 50 %-igen Natronlauge ein pH-Wert von 8,4 eingestellt. Bei 80 °C wurde im Verlauf von 4 Stunden eine Lösung von 42,04 g (1,0 mol) Cyanamid gelöst in 42 g Wasser zudosiert. Die Nachreaktion erfolgte für eine weitere Stunde bei 80 °C. Die erhaltene Suspension wurde auf 20 °C abgekühlt, abfiltriert, mit Wasser gewaschen und bei 60 °C getrocknet. Es wurden 100,1 g N-(Aminoiminomethyl)-2-aminoethansäure mit einem Gehalt von 99,2 % erhalten. Die Ausbeute betrug 84,8 %.

Ein Pulverdiffraktogramm der erhaltenen Kristalle zeigte, dass ausschließlich Form A vorlag. Auch wenn N-(Aminoiminomethyl)-2-aminoethansäure durch Reaktion zwischen Glycin und Cyanamid generiert wird, lässt sich die anfallende Kristallform durch die Anwesenheit unterschiedlicher Konzentrationen von Calciumchlorid steuern.

### Beispiel 7 - Physikalisch-chemische Charakterisierung von N-(Aminoiminomethyl)-2-aminoethansäure der Form A und der Form B

### 7.1 Schmelz- bzw. Zersetzungspunkt

Zur Dynamischen Differential Scanning Calorimetry (DSC) wurde ein Gerät Mettler DSC 3+ mit 40 µl Aluminiumtiegel eingesetzt. Die Heizrate betrug 10 Kelvin pro Minute bei einem Temperaturbereich von 30 bis 350 °C. Jeweils ca. 1,4 mg der Produkte aus Beispiel 1 und 2 wurden in Aluminium-Tiegel eingewogen und bei Atmosphärendruck (960 mbar bei einer Höhenlage von 500 m über NN) vermessen.

Die Probe aus Beispiel 1 (= N-(Aminoiminomethyl)-2-aminoethansäure der Form A) zeigte einen onset (Wendepunkt der Schmelzkurve projiziert auf die Basislinie) von 280,5 °C und eine Peaktemperatur der Schmelzkurve von 286,3 °C. Die gesamte endotherme Schmelzwärme betrug 887 J/g. Das Produkt verfärbte sich beim Schmelzen von weiß nach braun.

Die Probe aus Beispiel 2 (= N-(Aminoiminomethyl)-2-aminoethansäure Form B) wurde anlog vermessen. Sie zeigte einen onset von 272,5 °C und einen Peak bei 280,4 °C, die Schmelzwärme betrug 860 J/g, die Verfärbung war identisch.

Form B schmilzt demnach ca. 6 bis 8 Kelvin tiefer als Form A und hat eine um 27 J/g niedrigere Schmelzwärme bzw. eine um 27 J/g höhere Gitterenergie. Anders ausgedrückt werden für Form B 27 J/g weniger Energie benötigt als für Form A, um den energiegleichen Schmelzzustand zu erreichen. Form B stellt damit eine metastabile Kristallform bzw. ein unter normalen Druck- und Temperaturbedingungen energetisch höherliegendes Polymorph von N-(Aminoiminomethyl)-2-aminoethansäure dar.

### 7.2 Bestimmung der Wasserlöslichkeit

100 g Wasser von 5 °C wurden vorgelegt. Darin wurde das Produkt aus Beispiel 1 (= N-(Aminoiminomethyl)-2-aminoethansäure Form A) bis zur Sättigung gelöst und die gelöste Menge durch Rückwägung bestimmt. Dann wurde die Temperatur auf 20 °C erhöht und so viel der Probe zugegeben, bis wieder der Sättigungspunkt erreicht war. Dasselbe wurde bei weiteren Temperaturen, maximal bei 95 °C, wiederholt. Eine analoge Messung wurde mit dem Produkt aus Beispiel 2 (= N-(Aminoiminomethyl)-2-aminoethansäure Form B) durchgeführt. Die erhaltenen Löslichkeitsdaten für beide Produkte wurden graphisch in Figur 6 zusammengefasst.

Beide Kristallformen von N-(Aminoiminomethyl)-2-aminoethansäure lösen sich mit steigenden Temperatur besser in Wasser. Die erfindungsgemäße N-(Aminoiminomethyl)-2-aminoethansäure Form B löst sich bei jeder Temperatur um ca. 20 % besser als die bekannte Form A.

### 7.3 Bestimmung der Dichte

Kristalle von N-(Aminoiminomethyl)-2-aminoethansäure Form A aus Beispiel 1 wurden bei 20 °C in Tetrachlormethan eingebracht, wo sie an er Oberfläche schwammen. Durch tropfenweiser Zugabe von Dichlormethan wurde die Dichte des flüssigen Mediums so lange erniedrigt, bis die Kristalle gerade eben in der Flüssigkeit zu schweben kamen, ohne aufzusteigen und ohne auf den Boden abzusinken. Die Dichte der flüssigen Phase wurde im Pyknometer bestimmt. Es wurden 1,50 g/cm³ gemessen.

Analog wurde mit Kristallen der Form B aus Beispiel 2 verfahren. Die Dichte bei 20 °C wurde zu 1,41 g/cm³ bestimmt.

Form B hat demnach eine um 6 % geringere Dichte als Form A. Dies korreliert mit der oben bestimmten niedrigeren Gitterenergie von Form B. Die gemessenen Kristalldichten stimmen zudem mit den aus den jeweiligen Gitterkonstanten berechneten Röntgen-Kristalldichten überein.

### 7.4 Bestimmung des Staubanteils

Das Produkt aus Beispiel 1 wurde über ein Sieb mit Maschenweite 63 µm (entspricht 230 Mesh - Mesh-Größe) abgesiebt. Es wurden 46 Gew.-% Feinanteil erhalten. Analog wurde mit der aus polygonalen, rundlichen Kristallaggregaten bestehenden Probe aus Beispiel 2 verfahren. Hier wurde ein Feinanteil von unter 3 Gew.-% bestimmt. Staubarme, damit sicher handhabbare Materialien sollten einen Staubanteil (d.h. Kornanteil < 63 µm) von unter 10 % aufweisen. Das Produkt aus Beispiel 2 (N-(Aminoiminomethyl)-2-aminoethansäure der Kristallform B) erfüllt dies, während das Vergleichsbeispiel 1 (N-(Aminoiminomethyl)-2-aminoethansäure der Kristallform A) dies nicht erfüllt.

### 7.5 Bestimmung des Schüttwinkels

Das Produkt aus Beispiel 1, bestehend aus ineinander verfilzten nadeligen Kristallen, wurde mit einer Vorrichtung nach DIN ISO 4324 durch einen Trichter auf eine ebene Fläche geschüttet. Nach Entfernen des Trichters wurde der Böschungswinkel des erhaltenen Kegels mit einer Winkelmesseinrichtung bestimmt. Dieser betrug ca. 45 °. N-(Aminoimino-methyl)-2-aminoethansäure Form A zeigt demnach ein schlechtes Fließverhalten. Das körnige Produkt aus Beispiel 2 wurde anlog vermessen. Hier wurde ein Böschungswinkel von ca. 25 ° erhalten. N-(Aminoiminomethyl)-2-aminoethansäure Form B zeigt demnach ein hervorragendes Fließverhalten.

### 7.6 Bestimmung der Schüttdichte

Eine eingewogene Menge des Produkts aus Beispiel 1 wurde in einen Messzylinder gegeben und durch zweimaliges festes Aufklopfen auf den Labortisch partiell verdichtet. Aus der Füllhöhe des Messzylinders wurde die Schüttdichte zu 0,37 g/cm³ bestimmt. Analog wurde mit dem Produkt aus Beispiel 2 verfahren. Hier wurde eine Schüttdichte von 0,62 g/cm³ bestimmt. N-(Aminoiminomethyl)-2-aminoethansäure der Form B hat somit eine deutlich erhöhte Schüttdichte, was für Verpackung, Transport und Handhabung des Produkts vorteilhaft ist.

### 7.7 Thermische Stabilität von N-(Aminoiminomethyl)-2-aminoethansäure Form B

a) N-(Aminoiminomethyl)-2-aminoethansäure Form B aus Beispiel 2 wurde für 6 Stunden bei 120 °C in den Trockenschrank gestellt. Anschließend wurde mittels Röntgen-Pulver-Diffraktometrie die Kristallform bestimmt. Diese blieb unverändert reine Kristallform B.
b) N-(Aminoiminomethyl)-2-aminoethansäure Form B aus Beispiel 2 wurde mit 20 % Wasser angefeuchtet, für 6 Stunden bei 65 °C in einem geschlossenen Gefäß inkubiert, dann getrocknet. Das Röntgen-Pulver-Diffraktogramm zeigte keinerlei Veränderung, Form B blieb stabil.
c) N-(Aminoiminomethyl)-2-aminoethansäure Form B aus Beispiel 2 wurde zu einer 10 %-igen Suspension in Wasser angesetzt. Diese Suspension wurde 2 Stunden bei 80 °C gerührt. Dann wurde abgekühlt, der Feststoff abfiltriert und getrocknet. Die Röntgen-Pulver-Diffraktometrie ergab, dass ein Gemisch aus Kristallform A und B vorlag.
d) N-(Aminoiminomethyl)-2-aminoethansäure Form B aus Beispiel 2 wurde bei 80 °C in Wasser gelöst, durch Abkühlen der Lösung großteils wieder auskristallisiert, abfiltriert und getrocknet. Die Röntgen-Pulver-Diffraktometrie ergab reine Kristallform A.

N-(Aminoiminomethyl)-2-aminoethansäure Form B ist also in fester Form sehr beständig, hat jedoch die Tendenz, über die wässrige Lösung in Kristallform A überzugehen. Auch dieses Verhalten bestätigt die metastabile Kristallstruktur von Form B.

### Beispiel 8 - Synthese N-(Aminoiminomethyl)-2-aminoethansäure gemäß Stand der Technik in dem Calcium zugegen ist - DE 964 590 B

Anmerkung: Der gemäß DE 964 590 B eingesetzte Kalkstickstoff hat nur einen Gehalt von 53 %; dies entspricht 15,9 % N. In dem folgenden Beispiel wurde Kalkstickstoff mit einem Gehalt von 68,6 % eingesetzt; dies entspricht 24 % N. Die Einsatzmenge wurde entsprechend angepasst.

154,5 g technisches Calciumcyanamid mit einem Gehalt von 68,6 % CaNCN wurden in 800 g Wasser suspendiert. Bei 20 °C wurde eine Mischung aus 191,6 g einer 96 %igen Schwefelsäure und 300 g Wasser zudosiert, wobei Cyanamid in Lösung ging und Calciumsulfat aus der Lösung ausfiel und ein pH-Wert von 7,5 erhalten wurde. Calciumsulfat und sonstige unlösliche Bestandteile wurden abfiltriert und das Filtrat mit wenig Schwefelsäure auf pH 4,9 gestellt. Die erhaltene Lösung wurde unter vermindertem Druck bei ca. 10 mbar auf ein Gesamtvolumen von 200 cm³ eingedampft. Weiteres ausgefallenes Calciumsulfat wurde abfiltriert. Die erhaltene wässrige Cyanamid-Löung hatte einen Cyanamid-Gehalt von 26,4 % und einen Calciumgehalt von 0,56 g/Liter. (Anmerkung: entspricht einer Cyanamid-Ausbeute von 95 % und der Gips-Löslichkeit von 2,4 g/l).

Diese Cyanamid-Lösung wurde mit 30 g Glycin versetzt und mit 19,8 g einer 50 %-igen wässrigen Natronlauge auf pH 9,4 gestellt. Das Reaktionsgemisch wurde für 1,5 Stunden auf 95 °C erhitzt und dann über Nacht auf Raumtemperatur abgekühlt. Ausgefallene N-(Aminoiminomethyl)-2-aminoethansäure sowie mitentstandenes Dicyandiamid wurden abfiltriert, der Filterrückstand in 180 g Wasser aufgenommen, 2 Stunden bei 50 °C ausgelaugt, bei 50 °C filtriert und mit Wasser gewaschen. Nach Trocknung bei 60 °C wurden 38,4 g N-(Aminoiminomethyl)-2-aminoethansäure erhalten. Die Ausbeute betrug 82 % bezogen auf eingesetztes Glycin.

Eine Röntgen-Pulverdiffraktometrie ergab, dass N-(Aminoiminomethyl)-2-aminoethansäure ausschließlich in der Kristallform A entstanden war.

## Patentansprüche

1. Verfahren zur Herstellung einer thermodynamisch metastabilen Kristallmodifikation von N-(Aminoiminomethyl)-2-aminoethansäure, wobei die Kristallmodifikation im Röntgen-Pulver-Diffraktogramm der Kristallmodifikation bei Verwendung von Cu-Kα₁-Strahlung die stärksten Reflexbanden bei 2Θ = 20,2 ° und 23,3 ° und 23,8 ° und 25,3 ° bei einer Messgenauigkeit von +/- 0,2 ° zeigt, **dadurch gekennzeichnet, dass** Cyanamid und Glycin in einer wässrigen Calciumchlorid-haltigen Lösung umgesetzt werden, wobei die wässrige Lösung 5 bis 50 Gew.-% Calciumchlorid, bezogen auf das Reaktionsgemisch, enthält und die Kristallmodifikation aus dem Reaktionsgemisch der Umsetzung kristallisiert wird.

2. Verfahren nach Anspruch 1, **dadurch gekennzeichnet, dass** die wässrige Lösung neben Wasser ein weiteres Lösungsmittel aus der Gruppe der mit Wasser mischbaren organischen Lösemittel umfasst.

3. Verfahren nach einem der zuvor genannten Ansprüche, **dadurch gekennzeichnet, dass** das Glycin in der wässrigen Calciumchlorid-haltigen Lösung vorgelegt wird und das Cyanamid zugegeben wird.

4. Verfahren nach einem der zuvor genannten Ansprüche, **dadurch gekennzeichnet, dass** das Cyanamid als Feststoff oder in Form einer wässrigen Lösung in die Calciumchlorid-haltige Lösung oder das Reaktionsgemisch eingebracht wird.

5. Verfahren nach Anspruch 1, **dadurch gekennzeichnet, dass** die wässrige Lösung und/oder das Reaktionsgemisch neben Wasser kein weiteres Lösungsmittel umfasst.

6. Verfahren nach einem der zuvor genannten Ansprüche, **dadurch gekennzeichnet, dass** die Umsetzung bei Normaldruck in einem Temperaturbereich von 20 bis 100 °C erfolgt.

7. Verfahren nach einem der zuvor genannten Ansprüche, **dadurch gekennzeichnet, dass** die Umsetzung bei einem pH-Wert im Bereich von pH 7 bis 10 erfolgt.

8. Verfahren nach einem der zuvor genannten Ansprüche, **dadurch gekennzeichnet, dass** N-(Aminoiminomethyl)-2-aminoethansäure mit einer Abkühlrate im Bereich von 0,01 bis 5 K/min in einem Temperaturbereich von -40 bis 100 °C aus dem Reaktionsgemisch kristallisiert wird.

9. Verfahren nach einem der zuvor genannten Ansprüche, **dadurch gekennzeichnet, dass** die Kristallmodifikation die orthorhombische Raumgruppe P2₁2₁2₁ mit Z = 8 mit den Gitterkonstanten a = 7,7685 Å, b = 7,7683 Å und c = 17,4261 Å bei 105 Kelvin und einer Messgenauigkeit von +/- 0,001 Å aufweist.

10. Verwendung der thermodynamisch metastabilen Kristallmodifikation von N-(Aminoiminomethyl)-2-aminoethansäure gemäß Anspruch 1 zur Herstellung eines pharmazeutischen Wirkstoffs, zur Herstellung eines Nahrungsergänzungsmittels oder zur Herstellung eines Futtermittelzusatzes.

## Claims

1. Method for producing a thermodynamically metastable crystal modification of N-(aminoiminomethyl)-2-aminoacetic acid, wherein the crystal modification in the x-ray powder diffractogram of the crystal modification displays the strongest reflex bands at 2Θ = 20.2° and 23.3° and 23.8° and 25.3° at a measuring accuracy of +/- 0.2° when using Cu-Kα₁ radiation, **characterised in that** cyanamide and glycine are converted in an aqueous calcium chloride-containing solution, wherein the aqueous solution contains 5 to 50 wt.% calcium chloride, related to the reaction mixture, and the crystal modification is crystallised from the reaction mixture of the conversion.

2. Method according to claim 1, **characterised in that** the aqueous solution comprises a further solvent from the group of the organic solvents that can be mixed with water, in addition to water.

3. Method according to any one of the previous claims, **characterised in that** the glycine is provided in the aqueous, calcium chloride-containing solution and that the cyanamide is added.

4. Method according to any one of the previous claims, **characterised in that** the cyanamide is introduced into the calcium chloride-containing solution or the reaction mixture as a solid or in the form of an aqueous solution.

5. Method according to claim 1, **characterised in that** the aqueous solution and/or the reaction mixture comprises no further solvent apart from water.

6. Method according to any one of the previous claims, **characterised in that** the conversion takes place at normal pressure within a temperature range of 20 to 100°C.

7. Method according to any one of the previous claims, **characterised in that** the conversion takes place at a pH value within a range of 7 to 10.

8. Method according to any one of the previous claims, **characterised in that** N-(aminoiminomethyl)-2-aminoacetic acid is crystallised from the reaction mixture with a cooling rate within a range of 0.01 to 5 K/min within a temperature range of -40 to 100°C.

9. Method according to any one of the previous claims, **characterised in that** the crystal modification has the orthorhombic space group P2₁2₁2₁ with Z = 8 with the lattice constants a = 7.7685 Å, b = 7.7683 Å and c = 17.4261 Å at 105 Kelvin and a measuring accuracy of +/- 0.001 Å.

10. Use of the thermodynamically metastable crystal modification of N-(aminoiminomethyl)-2-aminoacetic acid according to claim 1 for producing a pharmaceutical active substance, for producing a food additive or for producing an animal feed additive.

## Revendications

1. Procédé de préparation d'une modification cristalline thermodynamiquement métastable de l'acide N-(aminoiminométhyl)-2-aminoéthanoïque, dans lequel la modification cristalline présente dans le diffractogramme de poudre aux rayons X de la modification cristalline, en utilisant un rayonnement Cu-K_{α1}, les bandes de réflexion les plus fortes à 2Θ = 20, 2 ° et 23,3 ° et 23,8 ° et 25,3 ° avec une précision de mesure de +/- 0,2 °, **caractérisé en ce que** le cyanamide et la glycine sont mis à réagir dans une solution aqueuse contenant du chlorure de calcium, dans lequel la solution aqueuse contient de 5 à 50 % en poids de chlorure de calcium, par rapport au mélange réactionnel, et la modification cristalline est cristallisée à partir du mélange réactionnel de la réaction.

2. Procédé selon la revendication 1, **caractérisé en ce que** la solution aqueuse comprend, outre de l'eau, un autre solvant choisi dans le groupe des solvants organiques miscibles à l'eau.

3. Procédé selon l'une quelconque des revendications précédentes, **caractérisé en ce que** la glycine est présentée dans la solution aqueuse contenant du chlorure de calcium et **en ce que** la cyanamide est ajouté.

4. Procédé selon l'une des revendications précédentes, **caractérisé en ce que** la cyanamide est introduite sous forme solide ou sous forme de solution aqueuse dans la solution contenant du chlorure de calcium ou dans le mélange réactionnel.

5. Procédé selon la revendication 1, **caractérisé en ce que** la solution aqueuse et/ou le mélange réactionnel ne comprend pas d'autre solvant que l'eau.

6. Procédé selon l'une des revendications précédentes, **caractérisé en ce que** la réaction est effectuée à pression normale dans une plage de température de 20 à 100 °C.

7. Procédé selon l'une des revendications précédentes, **caractérisé en ce que** la réaction a lieu à un pH compris entre 7 et 10.

8. Procédé selon l'une quelconque des revendications précédentes, **caractérisé en ce que** l'acide N-(aminoiminométhyl)-2-aminoéthanoïque est cristallisé à partir du mélange réactionnel à une vitesse de refroidissement comprise entre 0,01 et 5 K/min dans une plage de température de -40 à 100 °C.

9. Procédé selon l'une des revendications précédentes, **caractérisé en ce que** la modification cristalline présente le groupe spatial orthorhombique P2₁2₁2₁ avec Z = 8 avec les constantes de réseau a = 7,7685 Å, b = 7,7683 Å et c = 17,4261 Å à 105 Kelvin et une précision de mesure de +/- 0,001 Å.

10. Utilisation de la modification cristalline thermodynamiquement métastable de l'acide N-(aminoiminométhyl)-2-aminoéthano selon la revendication 1 pour la préparation d'un principe actif pharmaceutique, pour la préparation d'un complément alimentaire ou pour la préparation d'un additif alimentaire pour animaux.
